# EUROPÄISCHE PATENTSCHRIFT

(11) **EP 2 468 390 B1**
(45) Veröffentlichungstag und Bekanntmachung des Hinweises auf die Patenterteilung: **05.03.2014**
(21) Anmeldenummer: 11009861.3
(22) Anmeldetag: 15.12.2011
(51) Int. Cl.: B01F 1/00, B01F 5/10, B01F 15/00, G01F 25/00, A61M 1/14

(54) **Mischanlage**
Mixing assembly
Installation de mélange

(30) Priorität: 23.12.2010 DE 102010055781
(43) Veröffentlichungstag der Anmeldung: 27.06.2012
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- EP-A1- 0 491 981
- EP-A2- 0 147 806
- DE-A1- 19 702 213
- DE-B- 1 249 547
- DE-B3- 10 313 965
- FR-A1- 2 647 349
- US-A1- 2002 134 721
- US-A1- 2008 302 986

## Beschreibung

Die Erfindung betrifft eine Mischanlage zur Herstellung gebrauchsfähiger Lösungen aus flüssigen, pulvrigen, pastösen oder granulierten Konzentraten und einer hochreinen Flüssigkeit für den Einsatz als Konzentrat oder Spüllösung in der Medizintechnik, insbesondere für die Hämodialyse. Weitere Anwendungsgebiete für die mit der Mischanlage hergestellten Lösungen sind Purisolelösungen für den Einsatz während einer Blasenoperation oder einer postoperativen Blasenspülung, Peritoneallösungen für eine Bauchfell-Dialysebehandlung, Spüllösungen für chirurgische Eingriffe und Spüllösungen für Athroskopie, wobei diese Aufzählung nicht abschließend zu verstehen ist.

Die Mischanlage enthält eine Reinstwasserquelle, z.B. eine Umkehrosmose, die bevorzugt unter Verwendung eines Sterilfilters das von ihr erzeugte Permeat zu der Reinstwasserzulaufleitung des Mischgerätes führt, wobei das Mischgerät einen Rechner und einen Rezirkulationskreislauf enthält, in den eine Pumpe, eine Heizeinrichtung, ein Rohstoffbehälter, der vor Beginn des Mischvorgangs flüssiges, pastöses, pulvriges oder granuliertes Konzentrat enthält, und ein Fertiglösungsbehälter eingeschaltet sind, der vor Beginn des Mischvorgangs mit einem definierten Volumen des Reinstwasser befüllbar ist.

Es ist seit langem bekannt, gebrauchsfertige Lösungen zentral herzustellen und zu dem Verbraucher zu versenden, wobei dies mit hohen Frachtkosten verbunden ist.

Daneben sind dezentrale Mischanlagen bekannt, die in vielerlei Hinsicht Nachteile aufweisen. Sie sind unzureichend hinsichtlich der Produktgenauigkeit. Beispielsweise sind bei Dialysekonzentraten Natriumabweichungen von maximal +/- 2,5% erlaubt. Ferner sind sie hinsichtlich der Hygieneanforderungen unzureichend, wobei die Anforderungen bei der Mikrobiologie < 100 kBE und < 0,25 EU lauten. Die chemischen und thermischen Desinfektionsmaßnahmen sind teuer und aufwändig. Außerdem sind dezentrale Mischanlagen groß und auf fixe Tanks bzw. Behälter beschränkt.

Es kommt hinzu, dass die Verteilung bzw. Anwendung am Benutzungsort teuer bzw. aufwändig ist. Nach erfolgter Mischung ist die Lösung kalt und damit als Spüllösung nicht gebrauchsfertig.

US2002/134721 A1 offenbart eine Mischanlage zur Herstellung gebrauchsfähiger Lösungen für medizinische Zwecke, mit einer Reinstwasserquelle, die über eine Zulaufleitung mit einer Zulaufleitung eines Mischgerätes verbunden ist, wobei das Mischgerät einen Rechner und einen Rezirkulationskreislauf enthält, in den eine Pumpe, ein Rohstoffbehälter, der vor Beginn des Mischvorgangs flüssiges, pastöses oder pulveriges oder granuliertes Konzentrat enthält, und ein Fertiglösungsbehälter eingeschaltet sind, der vor Beginn des Mischvorgangs mit einem definierten Volumen des Reinstwassers befüllbar ist.

EP 0 147 806 A2, US2008/302986 A1 und DE 1249547 B offenbaren Prüfeinrichtungen für Durchflussmesser mit Messkammern, in denen der Füllstand ermittelt wird, wobei der ermittelte Wert mit dem Messwert wenigstens eines Flussmessers verglichen und dessen Messwert erforderlichenfalls korrigiert wird.

DE 19702213 A1 offenbart eine Vorrichtung zur Herstellung von Dialysierflüssigkeit, die Mittel zur Überwachung der Leitfähigkeit und der Temperatur der Fertiglösung aufweist.

FR2647349A1 offenbart eine Vorrichtung zur Herstellung einer medizinischen Flüssigkeit, die einen Rohstoffbehälter mit tangentialem Zulauf und Ablauf und wenigstens einen Druckluftanschluss aufweist, um Leitungen und Behälter zu entleeren.

EP 0 491 981 A1 offenbart einen Rohstoffbehälter mit einem verschließbaren Deckel mit einem inneren kegelförmigen Ansatz.

Der vorliegenden Erfindung liegt die Aufgabe zugrunde, wenigstens einige der oben genannten Nachteile des Standes der Technik zu vermeiden. Insbesondere besteht eine Aufgabe darin, eine Mischanlage anzugeben, bei der unter Verwendung von hochreiner, keimfreier Flüssigkeit eine hochgenaue Zudosierung der Flüssigkeit gewährleistet ist und bei der beim Mischvorgang die Dichte der Flüssigkeit online gemessen werden kann.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, dass in der Zulaufleitung der Reinstwasserquelle oder in der Zulaufleitung des Mischgerätes wenigstens ein mit einem zugehörigen Rechner verbundener Flussmesser eingeschaltet ist, und dass stromabwärts davon eine volumetrische Messkammer angeordnet ist, in der der Füllstand der Messkammer ermittelbar ist, wobei der ermittelte Wert durch den Rechner des Mischgerätes mit dem Messwert des wenigstens einen Flussmessers verglichen und dessen Messwert erforderlichenfalls korrigiert wird.

Erst wenn die Angabe des wenigstens einen Flussmessers über die Durchflussmenge mit dem bekannten Messwert der Messkammer in Einklang steht und der Flussmesser kalibriert ist, wird die für den jeweiligen Mischvorgang vorgesehene Reinstwassermenge unter Berücksichtigung der zum Zwecke der Kalibrierung bereits zugelaufenen Wassermenge in den Rezirkulationskreislauf eingefüllt, so dass diese Zudosierung mit höchster Genauigkeit erfolgt.

Dabei ist bevorzugt, dass zwei Flussmesser hintereinander angeordnet sind, die als Betriebsflussmesser und Überwachungsflussmesser redundant arbeiten und jeweils von zugehörigen Rechnern überwacht bzw. kalibriert werden. Hierdurch wird die hochgenaue Zudosierung im höchsten Maße ausfallsicher.

Die Zulaufleitung des Mischgerätes führt zu einem Füllventil des Zirkulationskreislaufs. Die Messkammer kann grundsätzlich in Strömungsrichtung vor dem Füllventil angeordnet sein, es ist jedoch bevorzugt, dass die Messkammer in den Rezirkulationskreislauf eingeschaltet ist. Dies ist insbesondere dann der Fall, wenn die Messkammer so ausgestattet ist, dass sie nicht nur den Füllstand erfasst (d.h. den Zeitpunkt des Erreichens eines festliegendes Füllniveaus in der Messkammer erfasst, wobei das Volumen der zuführenden Leitung und der Messkammer bekannt sind), sondern auch derart, dass in der Messkammer die Dichte der durchströmenden Flüssigkeit erfasst werden kann. Hierzu kann an der Außenwand der Messkammer ein Ultraschalldichtesensor mit seinen zugehörigen Komponenten angeordnet sein.

In einer bevorzugten Ausgestaltung der Erfindung hat die Messkammer einen oberen transparenten Abschnitt, der mit optoelektronischen Mitteln versehen ist, mit der der Füllstand, bzw. das Erreichen einer bestimmten Füllhöhe, messbar ist. Alternativ hierzu kann in die Messkammer ein kapazitiver Füllstandssensor eingesetzt sein.

Außerdem wird vorgeschlagen, dass die Messkammer mit einer elektrolytischen Ozonzelle versehen sein kann, wobei es sich um eine kostengünstige Desinfektionsmaßnahme handelt.

Das Mischgerät enthält ferner an geeigneten Stellen Mittel zur Überwachung der Leitfähigkeit der hergestellten Flüssigkeit und ihrer Temperatur. An den Rezirkulationskreislauf ist ein Transferventil angeschlossen, so dass die gebrauchsfähig erzeugte Lösung zu Spülzwecken direkt einsetzbar ist oder in weitere Lagerbehälter abgefüllt wird.

Der Messzylinder hat ein bekanntes Volumen (ebenso wie die zuführende Leitung) und einen sich nach oben verjüngenden zylindrischen Endabschnitt, der bevorzugt transparent ist. An diesem transparenten Ende kann elektronisch der Füllstand ermittelt und über den Rechner der Mischanlage mit den Flussmesserwerten verglichen werden.

Das Gerät ermöglicht die Online-Überprüfung der Lösung mittels Dichte-, Leitfähigkeits- und Temperaturmessungen während jedes Mischvorgangs. Die erzeugte Lösung, die auf einer vorgegebenen Temperatur gehalten wird, lässt sich auf einfache Weise verteilen.

Nach einem weiteren Vorschlag der Erfindung ist der Rezirkulationskreislauf mit wenigstens einem Druckluftanschluss versehen, um die fertig gemischte Lösung mittels der Druckluftzuführung zu fördern und zu entleeren.

Als Rohstoffbehälter können Wegwerfbehälter oder auch wiederbefüllbare Behälter verwendet werden. Nach einem besonders vorteilhaften Vorschlag enthält der Rohstoffbehälter einen unteren tangentialen Zulauf und einen oberen tangentialen Ablauf, wobei außerdem ein zusätzlicher unterer tangentialer Ablauf kleineren Durchmessers vorgesehen ist, durch den Flüssigkeit aus dem Behälter über eine außerhalb des Behälters angeordnete Leitung in die obere Ablaufleitung eingeführt wird, wenn der Behälterinhalt so verklumpt und verstopft ist, dass (zunächst) kein Ablauf durch die obere tangentiale Auslauföffnung erfolgen kann.

Weiter kann vorgesehen sein, dass der Rohstoffbehälter einen verschließbaren Deckel mit einem nach innen ragenden, mittigen, kegelförmigen Ansatz aufweist, der den Freiraum in dem Behälter während des Mischvorgangs ausfüllt. Dies führt zu einer sehr intensiven Vermischung in dem Behälter, wobei sich infolge des kegelförmigen Deckels weniger Feststoffe an der Wand des Behälters ablagern.

Als Fertiglösungsbehälter (die zunächst mit Reinstwasser befüllt werden) können mobile oder fixe Tanks bzw. Beutel verwendet werden. Der Tank bzw. Beutel kann über die tiefste Stelle befüllt und entleert werden. Es kann aber auch vorgesehen sein, dass die Flüssigkeitszirkulation über die höchste Stelle des Tanks zugeführt und über die niedrigste Stelle des Tanks wieder in den Zirkulationskreislauf eintritt.

Bevorzugt ist ferner, dass bei der Verbindung einer Umkehrosmose mit dem Mischgerät zu einer Einheit eine gemeinsame Steuerung vorgesehen ist.

Weitere Merkmale und Einzelheiten der Erfindung ergeben sich aus der nachfolgenden Beschreibung sowie anhand der beigefügten Figuren.

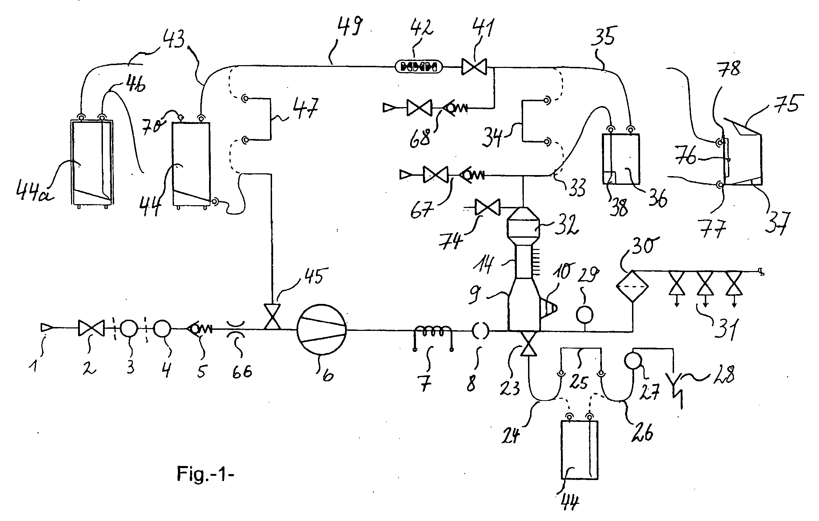

Figur 1 zeigt den Flüssigkeitsverlauf über die Reinstwasserzulaufleitung (1), das Wassereingangsventil (2), die Flussmesser (3, 4) mit den Beruhigungsstrecken (65), zur Verbesserung der Flussmessergenauigkeit, das Rückschlagventil (5) und die wahlweise einsetzbare Konstantflussdrossel (66), die zur Erhöhung der Flussmessergenauigkeit beiträgt. Zur Validierung der Flussmesser (3,4) wird Flüssigkeit bei geöffneten Wassereingangsventil (2) über Pumpe (6), Heizer (7), Leitfähigkeitsmessung (8) in die zuvor entleerte Messkammer (9) tangential gefördert. Die Leitfähigkeits-messung (8) kann in beliebigen Stellen des Zirkulationskreislaufes angeordnet sein. Bei bekanntem Volumen der Messkammer (9) wird die Flüssigkeit entweder mittels Optoelektronik in der transparenten Messtrecke (14) ermittelt und durch den Rechner der Mischanlage (hier nicht dargestellt) mit den Flussmesserergebnissen (3/4) verglichen. Dabei ist die Messkammer (9) nicht auf den gezeichneten Einbauort beschränkt, sondern kann mit Vorteil auch stromaufwärts des Zirkulations- und Füllventiles (45) eingebaut werden. Ebenso besteht neben der optoelektronischen Messung auch die Möglichkeit kapazitiv oder über einen Näherungsschalter den Füllstand abzufragen. Alternativ zur Messkammer (9) kann bei bekannter Tank- oder Beutelgeometrie die Validierung der Flussmeser (3/4) auch mittels eines Füllstandssensores im unteren Bereich des Tanks (44) oder des Folienbeutels (54) im Behälter (59) verwendet werden. Der untere Bereich des Tanks bzw. Beutels wird gewählt, weil bereits bei geringen zugeführten Volumina ein Aussage zur Genauigkeit der Flussmesser (3,4) zu treffen ist.

Bei Übereinstimmung der Messwerte der optoelektronischen Messung und der Flussmesser wird Ventil (45) geöffnet und der Tank bzw. das Behältnis (44/44a) mit der voreingestellten und durch die Flussmesser (3,4) überwachten Menge gefüllt. Dabei arbeiten Flussmesser (3) als Betriebsflussmesser und Flussmesser (4) als Überwachungsflussmesser redundant. Der Rechner der Mischanlage enthält einen Betriebs- und einen Schutzsystemrechner. Diese werten die Flussmesserergebnisse (3/4) unabhängig voneinander aus und vergleichen die Ergebnisse während des gesamten Füllprozesses. Die Flussmesser (3,4) können dabei auch in einer vorgeschalteten Reinstwasseranlage eingebaut sein, die mit der Mischeinheit eine Einheit bildet.

Während des Füllvorganges bleibt Ventil (41) geschlossen, so dass keine weitere Flüssigkeit über die Messkammer (9) und den angeschlosssenen Rohstoffbehälter (37,38) fließt.

Vorzugsweise ist das Behältnis (44) ein stationärer Tank mit Tankentlüftung (70) und Behältnis (44a) vorzugsweise ein Behältnis mit Folienbeutel (54). Bei Verwendung eines stationären Tankes (44) besteht die Möglichkeit, den Teil der Zirkulationsstrecke (43) mit einem Sprühkopf abzuschließen.

Nach abgeschlossener Füllung des Behälters (44) wird die Pumpe (6) eingeschaltet und Permeat über die Rohstoffbehälter (36/37) bzw. die angeschlossenen Leitungen (33/35) zirkuliert. Dabei wird das Ventil (41) geöffnet und die Lösung über den statischen Mischer (42) so vermischt, dass zwei sich kreuzende Teilströme entstehen. Vorzugsweise besteht der statische Mischer aus zwei symentrisch gedrehten Profilen, die in ein Rohr oder wahlweise im gesamten Zirkulationskreis eingebaut sein können. Die so zirkulierte Flüssigkeit wird über die Zulaufleitung (49) zum Fertiglösungsbehälter (44) zirkuliert. Während der Zirkulation wird je nach Erfordernis die Heizung (7) zugeschaltet und Temperatur- und Leitfähigkeit über Sensor (8) überwacht. Wobei Heizer (7) auch in der vorgeschalteten Reinstwasseranlage eingebaut werden kann. In diesem Fall wird die Mischanlage mit bereits vorgewärmtem Reinstwasser gefüllt.

Ebenso ist eine Überwachung der Dichte über eine Ultraschallmessung, eine Kombination aus Druck und Füllstand, oder eine annähernde Dichtemessung über einen kapazitiven Füllstandssensor möglich. Es besteht auch die Möglichkeit, über ein im Zirkulationskreis eingebautes Probeentnahmeventil (nicht dargestellt) eine manuelle Probe für Laboruntersuchung zu entnehmen. Denkbar sind auch Qualitätsnachweise über Ionensensitive Onlinemessungen.

Die Pumpe (6) kann dabei über Druckaufnehmer (29) so gesteuert bzw. geregelt werden, dass kein unzulässiger Systemdruck insbesondere bei den Behältnissen (36/37) entsteht. Ebenso kann über den Druckaufnehmer (29) die Dichtheit des Systems und der Leitungen der angeschlossenen Behältnisse getestet werden.

Vorzugsweise erhalten die Rohstoffbehälter (36/37) einen Barcode zur Identifikation des Inhaltes, der Charge, der Dichte, Leitfähigkeit usw. Diese Angaben werden vor dem Mischvorgang eingelesen und mit den ermittelten Messergebnissen verglichen. Diese Dokumentation dient der Qualitätssicherung.

Das Behältnis (44a) wird über Anschluss (43), über die Saugleitung (46) und Ventil (45) zirkuliert. Vorzugsweise ist dieses Behältnis ein beutelartiger Kunststoffbehälter oder Folienbeutel.

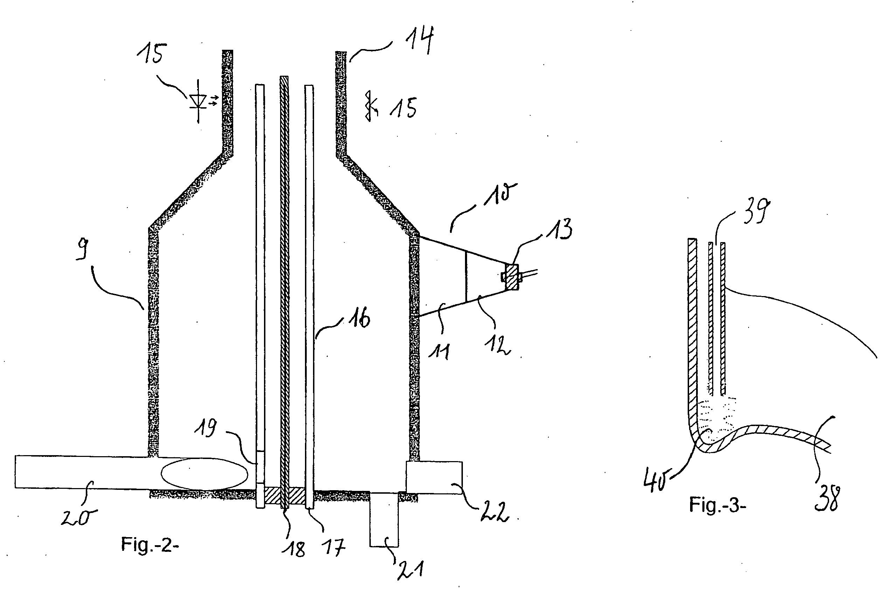

Der Rohstoffbehältern (36) wird vorzugsweise als Wegwerfeinheit geliefert, die Ausführung ist vorzugsweise ein Folienbeutel oder ein tankförmiges Gebilde gefüllt mit nassem, pulvrigem, granuliertem oder flüssigem Rohstoff. Bei einer tankförmigen Ausführung erfolgt die Zuführung in das Behältnis (36) wie Figur 3 zeigt, über die untere Anschlussleitung (33/39) und einen faltenbalgartigen Auslauf (40) der den Anschluss (39) fest auf den Tankboden drückt. Dabei ist der Tank nach innen gewölbt, sodass für die spätere Entleerung keine Restmengen zurückbleiben.

Behältnis (37) ist ein wiederbefüllbarer Rohrstoffbehälter mit geradem, schrägem oder konischem Boden und einem unteren tangentialen Zulauf (77), der von vorne gesehen unten rechts mit einem großen Durchmesser z.B. 32 mm angeordnet ist.

Der Behälter weist einen oberen tangentialen Ablauf (78), der von vorne gesehen links oben angeordnet ist, und einen Durchmesser von z. B. 15 mm hat und einen weiteren unteren tangentialen Ablauf (76) der von vorne gesehen unten links angeordnet ist und einen Durchmesser von z. B. 20 mm aufweist. Das Behältnis (37) hat einen verschließbaren Deckel (75) mit Kegelspitze, die den Freiraum des Rotationsparaboloiden während des Mischvorganges im Behälter ausfüllt und dadurch eine sehr hohe Intensität der Vermischung liefert.

Der untere Abfluss (76) dient dem zusätzlichen Austrag des eingefüllten Rohstoffes bei geringen Durchflüssen durch den Behälter (37) zu Beginn des Mischvorganges, z.B. bei Verklumpung, und wird an der Außenwand des Behälters (37) so angeordnet, dass er am oberen Ende in den Ablauf (78) einmündet.

Nach fertiggestellter Mischung wird Ventil (41) geschlossen und Druckluft über den Druckluftsteueranschluss (68) eingeblasen. Dabei wird der Rohstoffbehälter (36/37) die Zirkulationsstrecke stromaufwärts über Ventil (45) einschließlich der Messkammer (9) in das Behältnis (44/44a) entleert. Nach erfolgter Entleerung des Rohstoffbehälters (36/37) können die Anschlüsse (33/35) auf die Kurzschlussbrücke (34) zurückgesteckt werden, wobei mit Vorteil die Steckposition der Anschlussleitungen (35/33) mittels elektronischer Kontakte an der Kurzschlussbrücke (34) abgefragt werden. Die Abfrage der flexiblen Anschlussleitung lässt sich auch an den Kurzschlussbrücken (25/47) realisieren.

Zum Transfer wird das fertig gemischte Produkt aus Behältnis (44/44a)) über Pumpe (6) und Transferfilter (30) und Ventilen (31) zu den Verbrauchsstellen oder Lagertanks gefördert. Der Transfer bzw. die Entleerung kann sowohl über die Pumpe als auch bei Behälter (44a) mittels Lufteinspeisung am Anschluss (43) erfolgen.

Um nach der Mischung und dem Transfer hygienisch einwandfreie Verhältnisse wiederherzustellen, werden die Anschlüsse (33/35) auf die Kurzschlussbrücke (34) zurückgesteckt. Bei Verwendung des Behälters (44a) sind ebenfalls die Anschlüsse (43/46) auf Kurzschlussbrücke (47) zurückzustecken. Das System wird durch Öffnen des Ventiles (2) mit Wasser wieder gefüllt. Zur Entlüftung kann am oberen Ende des elektrolytischen Ozongenerators ein Entlüftungsventil (74) eingebaut werden, wobei die Lage des Entlüftungsventiles (74) nicht auf diesem Einbauort beschränkt ist. Durch Zirkulation der Flüssigkeit über Pumpe (6) und zuschalten der elektrolytischen Ozonzelle (32) werden dem System Sauerstoffradikale zur Dekontamination zugeführt. Dabei besteht die Ozonzelle aus zwei äußeren Kathoden und einer innenliegenden vorzugsweise aus graphithaltigem Material bestehenden ringförmigen Anode. Die im Kreislauf befindliche Flüssigkeit wird durch Öffnen des Ventiles (23) zum Abfluss (28) ausgespült.

### Figur 2

Alternativ zu der optoelektronischen Messung am oberen transparenten Ende der Mischkammer kann ein kapazitiver Füllstandsensor (16) eingesetzt werden, dessen Elektroden von einem äußeren Metallrohr (17) und einem koaxial darin angeordnetem isolierten Draht (18) oder Stab gebildet werden.

Das Rohr ist auf der einen Seite offen und hat mindestens eine seitliche Öffnung (19). Mit ansteigendem Füllstand wird der Innenraum zwischen Rohr und Stab mit Flüssigkeit aufgefüllt.

Insgesamt verhält sich ein solcher Sensor wie ein Kondensator, dessen Kapazität ausgehend von einem Anfangswert linear mit dem Füllstand zunimmt. Zur Signalgewinnung eignet sich wegen der Einfachheit z.B. eine Oszillatorschaltung, die eine Schwingung mit einer der Kapazität proportionalen Periodendauer erzeugt. Dieses Signal kann mit einem Mikroprozessor der Mischanlage leicht ausgewertet werden. Dabei können die Signale des kapazitiven Sensors (16) mit den Flussmesserwerten (3,4) mittels Rechner der Mischanlage ausgewertet bzw. verglichen werden.

Es hat sich gezeigt, dass mit diesem Verfahren auch eine Dichtemessung möglich ist.

Allerdings ist parallel zu der Kapazität des kapazitiven Sensors ein Verlustwiderstand wirksam, der von der Leitfähigkeit des Wassers abhängt. Dieser bleibt vernachlässigbar, wenn eine ausreichend hohe Messfrequenz benutzt wird.

Bei Flüssigkeiten mit höherer Leitfähigkeit kann dieser Verlustwiderstand trotz höheren Messfrequenzen zu erheblichen Messfehlern führen. Deshalb ist bei höheren Leitfähigkeiten diese Art der Dichtemessung nur eingeschränkt möglich.

Zur Lösung wird hier ein Ultraschallmessverfahren vorgeschlagen. Der Ultraschallsensor (10) kann dabei online zur Messung der spezifischen Dichte herangezogen werden. Der Messkörper (10) erhält einen einzigen Ultraschallsensor (13) in Form einer Piezoscheibe zur Erzeugung einer Ultraschallwelle die auf die Oberfläche des zu messenden Fluids gerichtet ist und zum Empfang sowohl eines Referenzechos als auch eines Nutzechos dient. Dazu ist der Ultraschallsensor (10) in zwei Kegelschnittflächen (11,12) angeordnet. Die Grenzschicht zwischen diesen beiden Kegelschnittflächen dient zur Erzeugung des Referenzechos. Das Nutzecho wird durch die Fluidoberfläche gebildet. Zur Unterdrückung von Störechos und optimaler Einkopplung des Nutzechos in das Fluid, sind die Kegelschnittflächen (11) vorzugsweise aus Edelstahl und (12) aus Kunststoff gebildet. Das online ermittelte Dichte Messergebnis kann über einen Drucker neben Angabe der Leitfähigkeit und der Temperatur als Messwertprotokoll ausgedruckt werden.

Bei der Kapazitiven- wie auch der Ultraschalldichtemessung wird die Dichte dabei jeweils relativ zur Dichte des Reinstwassers ermittelt. Durch diese Messmethode kann die Dichtemesseung einfacher gestaltet werden, weil zunächst vor jedem Mischvorgang die Dichtemesseinheit mit der Dichte des Reinstwassers kalibriert wird, und bei der Online Messung der Erwartungswert der Fertiglösung als bekannt vorausgesetzt wird.

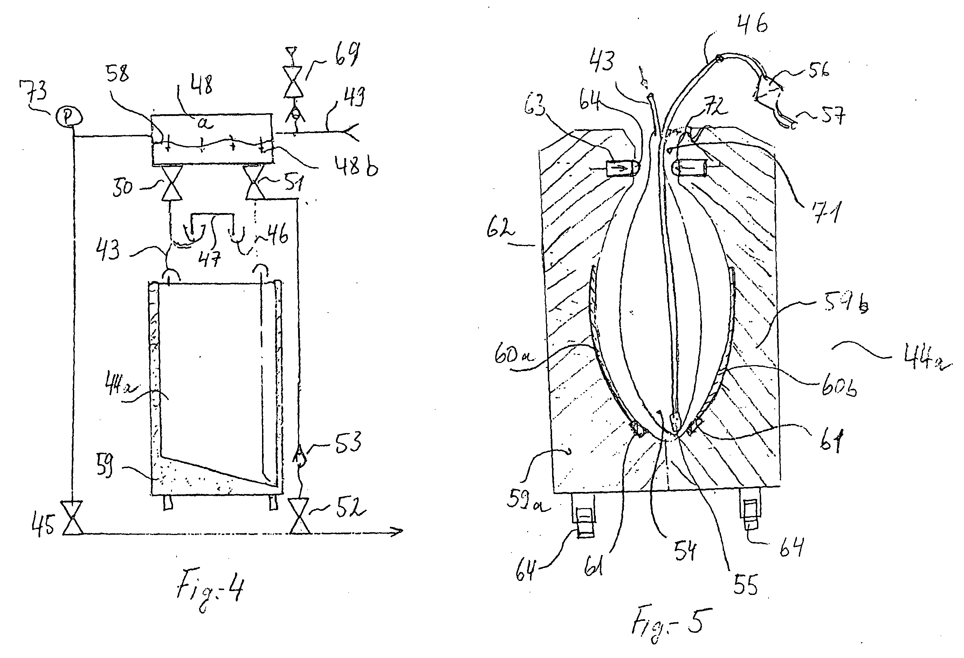

Figur 4 zeigt die Herstellung hochsteriler Lösung über einen Sterilfilter (48a/b) und ein vorzugsweise beutelartiges Behältnis (44a), das in einem isoliertem Schalenkörper (49) eingebettet ist. Die Befüllung des Behältnisses erfolgt wie bereits beschrieben über Ventil (45) und Filtermembran (58). Dabei kann wahlweise über Ventil (51) nach unten oder Ventil (50) von oben befüllt werden. Während des Mischvorganges wird Permeat über die Zulaufleitung (49) über den Primärraum des Sterilfilters (48a) gefördert, wobei Pumpe (6) die Zirkulation übernimmt. Gleichzeitig wird ein kleiner Nebenstrom über Ventil (52/53) mitzirkuliert. Mit dieser Methode ist es möglich das Behältnis (44a) steril zu befüllen und trotzdem über das Behältnis (44a) zu zirkulieren. Ein weiterer Vorteil ist, dass der Primärraum des Sterilfilters (48a) permanent überströmt wird und ein Blockieren durch nicht gelöste Rohstoffe verhindert wird.

Zur Validierung des Sterilfilters (48) wird mittels Lufteintrag über Zuluft (69) die Flüssigkeit im Primärraum (48a) und durch das zunächst geöffnete Ventil (45) verdrängt. Bei geschlossenem Ventilen (45/41) und geöffneten Ventilen (51/52/23) wird bei laufender Pumpe (6) Unterdruck auf der Primärseite des Sterilfilters (48a) angelegt. Da der Filter luftundurchlässig ist, ist der über den Druckaufnehmer (73) ermittelte Druck ein Maß für die Filterdichtheit, weil bei einer Filterleckage ein schneller Druckabbau von Primär- zur Sekundärseite erfolgen würde. Der Filtertest ist auch mit Überdruck auf der Primärseite (48a) durchzuführen.

Figur 5 zeigt einen Beutelporter mit zwei isolierten Schalen (59a/b), die durch eine geeignete Konstruktion auseinanderklappbar sind. Der Porter steht auf Rollen (64). In einen oberen Schlitz (71) wird der Leerbeutel eingeschoben und an einem Befestigungshaken (72) eingerastet. Nach erfolgter Befüllung, Mischung und Temperierung der Lösung über die Leitungen (43/46) schmiegt sich die Beuteloberfläche den Konturen der isolierten Schalen (59a/b) so an, dass bei Bedarf die innenliegenden Heizmatten (60a/b) einen Temperaturverlust ausgleichen können. Um Ausfällungen des Rohstoffes im Beutel (54) zu vermeiden, können mit Vorteil Ultraschallsensoren (61) oder eine andere vibrationstechnische mechanische Beeinflussung der Flüssigkeit im Beutelporter (62) angebracht werden. Zum Nachheizen der gebrauchsfertigen Lösung besteht auch die Möglichkeit, die Anschlussleitung (46) des Folienbeutels (54) in eine Schlauchheizeinheit einzulegen, um während des Flüssigkeitstransportes zu temperieren.

Zum Transfer wird an den Anschluss (43) Druckluft mit einer erforderlichen Sicherheitseinrichtung in Form eines Druckreglers, Überdruckventil und Schnelldruckentlüftung am Beutelporter installiert. Während des Transfers werden die Dichtzylinder (63) mit den Dichtlippen (64) mechanisch oder elektrisch so verriegelt, dass eine Diskonnektion bzw. Ruptur des Behältnisses (54) verhindert wird. Durch das Einbringen steriler Luft über Anschluss (43) wird die gebrauchsfertige, erwärmte Flüssigkeit über Anschluss (46), Einmalsterilfilter (56) und Überleitsystem (57) zum Einsatzort gebracht. Mit Vorteil wird dabei der Einsatz eines hydrophoben Sterilfilters (56) vorgeschlagen. Damit wird verhindert, dass Luft über das Überleitsystem (57) zum Patienten gelangt. Anstelle des Lufteintrages in den Anschluss (43) könnte auch ein Doppelbeutel mit äußerer Umhüllung so eingesetzt werden, dass die äußere Beutelschicht zum Lufteintrag oder auch als Reservoir für gebrauchte Lösung genutzt werden könnten.

Nicht verbrauchte und nicht mehr benötigte fertiggemischte Lösung kann über die Anschlüsse (24/25/26/27/28) entleert werden. Die Entleerung erfolgt wie bereits für die Behältnisse (36/37) beschrieben. Luftdetektor (27) zeigt das Ende des Entleerungsvorganges an.

Alternativ zum Beutelporter (62) besteht auch die Möglichkeit, einen fahrbaren zylindrischen Glasbehälter einzusetzen, da Glas sowohl hygienisch einfach zu reinigen ist und thermisch gut isoliert. Es besteht auch die Möglichkeit, den Rohstoff direkt in den Beutel Fertiglösung (54) abzufüllen und so ohne Disposables (38) eine gebrauchsfertige Lösung herzustellen.

Es wird betont, dass die Erfindung nicht auf die beschriebenen und dargestellten Ausführungsformen beschränkt ist. Vielmehr sind alle offenbarten Merkmale der Ausführungsformen auch einzeln untereinander anders als oben beschrieben miteinander kombinierbar.

**Legende**

| | |
|---|---|
| 1. | Reinstwasserzulaufleitung |
| 2. | Wassereingangsventil |
| 3. | Flussmesser 1 |
| 4. | Flussmesser 2 |
| 5. | Rückschlagventil |
| 6. | Zirkulationspumpe (Drehzahlgeregelt oder Druckgesteuert) |
| 7. | Heizung |
| 8. | Leitfähigkeitsmessung mit Temperaturüberwachung |
| 9. | Messkammer |
| 10. | Ultraschalldichtesensor |
| 11. | Material 2 |
| 12. | Material 1 |
| 13. | Piezoscheibe |
| 14. | Transparente Messstrecke |
| 15. | Elektronische Messeinrichtung |
| 16. | Kapazitiver Füllstandsensor |
| 17. | Rohrförmige Aussenelektrode |
| 18. | Innenelektrode mit Isolierung |
| 19. | Seitliche Zuflussöffnung des kapazitiven Sensors |
| 20. | Tangentialer Einlauf |
| 21. | Abflussbohrung für Trenage |
| 22. | Transferleitung |
| 23. | Drainageventil/Verwurfventil |
| 24. | Obere Entleerungsanschluss |
| 25. | Kurzschlussbrücke Entleerung |
| 26. | Untere Entleerungsanschluss |
| 27. | Leererkennungssensor |
| 28. | Trenage |
| 29. | Druckaufnehmer |
| 30. | Transferfilter |
| 31. | Transferventil |
| 32. | Elektrolytische Ozonzelle |
| 33. | Anschluss untere Zuflussleitung Rohstoffbehälter |
| 34. | Kurzschlussbrücke |
| 35. | Anschluss obere Zuflussleitung Rohstoffbehälter |
| 36. | Mobile Rohstoffbehälter (Wegwerfbehälter) |
| 37. | Stationärer Rohstoffbehälter mit Deckel und unteren tangetialen Einlauf |
| 38. | Sichtausschnitt des Salzbehälters |
| 39. | Untere Zuflussleitung |
| 40. | Faltenbalgartiger Auslauf |
| 41. | Entleersperreventil Rohstoffbehälter |
| 42. | Statischer Mischer |
| 43. | Oberer Zulaufanschluss Behältnis Fertiglösung |
| 44. | /a Tank-/Behältnis-/Folienbeutel für Fertiglösung |
| 45. | Zirkulation- und Füllventil |
| 46. | Sauganschluss Fertiglösungsbehälter |
| 47. | Kurzschlussbrücke Fertiglösungsbehälter |
| 48. | Sterilfilter (a Primärseite, b Sekundärseite) |
| 49. | Zulaufleitung zu den Fertiglösungsbehälter |
| 50. | Füllventil Fertiglösungsbehälter |
| 51. | Mischventil Fertiglösungsbehälter |
| 52. | Zirkulationsventil Fertiglösungsbehälter |
| 53. | RS-Ventil Zirkulation Fertiglösung |
| 54. | Beutel Fertiglösung |
| 55. | Saug- und Ausströmanschluss |
| 56. | Einmalsterilfilter |
| 57. | Überleitsystem zum Patienten |
| 58. | Filtermembran |
| 59. | Isolierte Schalenkörper |
| 60. | Heizmattenauskleidung |
| 61. | Ultraschall- bzw. Vibrationsaktoren |
| 62. | Beutelporter bzw. Porter für Fertiglösung |
| 63. | Dichtzelinder |
| 64. | Dichtlippen |
| 66. | Konstantflussdrossel |
| 67. | Druckluftsteueranschluss |
| 68. | Druckluftsteueranschluss |
| 69. | Druckluftsteueranschluss |
| 70. | Tankentlüftung |
| 71. | Oberer Schlitz Beutelporter |
| 72. | Befestigungshaken Beutelporter |
| 73. | Druckaufnehmer Sterilfilter |
| 74. | Entlüftungsventil |
| 75. | Deckel mit Kegelspitze |
| 76. | Ablauf unten tangential kleinerer Durchmesser von vorne unten links |
| 77. | Zulauf unten tangential großer Durchmesser von vorne unten rechts |
| 78. | Ablauf oben tangential kleinster Durchmesser von vorne links oben |

## Patentansprüche

1. Mischanlage zur Herstellung gebrauchsfähiger Lösungen für medizinische Zwecke mit einer Reinstwasserquelle, die über eine Zulaufleitung (1) mit einer Zulaufleitung eines Mischgerätes verbunden ist,
wobei das Mischgerät einen Rechner und einen Rezirkulationskreislauf enthält, in den eine Pumpe (6), ein Rohstoffbehälter (36, 37), der vor Beginn des Mischvorgangs flüssiges, pastöses, pulveriges oder granuliertes Konzentrat enthält, und ein Fertiglösungsbehälter (44) eingeschaltet sind, der vor Beginn des Mischvorgangs mit einem definierten Volumen des Reinstwassers befüllbar ist,
**dadurch gekennzeichnet,**
**dass** in der Zulaufleitung (1) der Reinstwasserquelle oder des Mischgerätes wenigstens ein mit einem zugehörigen Rechner verbundener Flussmesser (3,4) eingeschaltet ist, dass stromabwärts davon eine volumetrische Messkammer (9) angeordnet ist, in der der Füllstand der Messkammer (9) ermittelt wird, wobei der ermittelte Wert durch den Rechner des Mischgerätes mit dem Messwert des wenigstens einen Flussmessers verglichen und dessen Messwert erforderlichenfalls korrigiert wird,
und **dass** die Messkammer (9) mit einem Ultraschalldichtesensor (10) zur Messung der Dichte der Flüssigkeit versehen ist.

2. Mischanlage nach Anspruch 1,
**dadurch gekennzeichnet,**
**dass** zwei Flussmesser (3,4) hintereinander angeordnet sind, die als Betriebsflussmesser und Überwachungsflussmesser redundant arbeiten und jeweils von zugehörigen Rechnern und dem Rechner des Mischgerätes überwacht werden.

3. Mischanlage nach Anspruch 1 oder 2,
**dadurch gekennzeichnet,**
**dass** die Messkammer (9) einen oberen transparenten Abschnitt hat, an dem der Füllstand mit dort angeordneten optoelektronischen Mitteln erfassbar ist.

4. Mischanlage nach Anspruch 1 oder 2,
**dadurch gekennzeichnet,**
**dass** in die Messkammer (9) ein kapazitiver Füllstandsensor (16) eingesetzt ist.

5. Mischanlage nach einem der Ansprüche 1 bis 4,
**dadurch gekennzeichnet,**
**dass** die Messkammer mit einer elektrolytischen Ozonzelle (32) versehen ist.

6. Mischanlage nach einem der Ansprüche 1 bis 5,
**dadurch gekennzeichnet,**
**dass** das Mischgerät ferner Mittel zur Überwachung der Leitfähigkeit der Fertiglösung aufweist.

7. Mischanlage nach einem der Ansprüche 1 bis 6,
**dadurch gekennzeichnet,**
**dass** das Mischgerät ferner Mittel zur Überwachung der Temperatur der Fertiglösung aufweist.

8. Mischanlage nach einem der Ansprüche 1 bis 7,
**dadurch gekennzeichnet,**
**dass** der Rohstoffbehälter (37) einen unteren tangentialen Zulauf (77) und einen oberen tangentialen Ablauf (78) hat.

9. Mischanlage nach Anspruch 8,
**dadurch gekennzeichnet,**
**dass** der Rohstoffbehälter einen weiteren unteren tangentialen Ablauf (76) kleineren Durchmessers hat, der über eine außerhalb des Rohstoffbehälters (37) angeordnete Leitung in die obere Ablaufleitung einmündet.

10. Mischanlage nach einem der Ansprüche 1 bis 9,
**dadurch gekennzeichnet,**
**dass** der Rohstoffbehälter (37) einen verschließbaren Deckel mit einem nach innen ragenden mittigen kegelförmigen Ansatz aufweist, der den Freiraum in dem Behälter (37) während des Mischvorgangs ausfüllt.

11. Mischanlage nach einem der Ansprüche 1 bis 10,
**dadurch gekennzeichnet,**
**dass** der Rezirkulationskreislauf mit wenigstens einem Druckluftanschluss versehen ist, um die Leitungen und die Behälter (36/37144,44a) zu entleeren.

## Claims

1. A mixing installation for producing usable solutions for medicinal purposes with a high purity water source, which is connected via a feed line (1) to a feed line of a mixing device, wherein the mixing device includes a data processor and a recirculation circuit, connected into which are a pump (6), a feedstock container (36, 37), which contains a liquid, pasty, pulverulent or granulated concentrate before the beginning of the mixing process, and a finished solution container (44), which is fillable before the beginning of the mixing process with a defined volume of the high purity water, **characterised in that** connected into the feed line (1) of the high purity water source or of the mixing device there is a flow meter (3, 4) connected to an associated data processor, that arranged downstream of it there is a volumetric measuring chamber (9), in which the filling level of the measuring chamber (9) is determined, wherein the determined value is compared by the data processor of the mixing device with the measured value from the at least one flow meter and its measured value is, if necessary, corrected, and that the measuring chamber (9) is provided with an ultrasonic density sensor (10) for measuring the density of the liquid.

2. A mixing installation as claimed in Claim 1, **characterised in that** two flow meters (3, 4) are arranged behind one another, which operate redundantly as an operational flow meter and a monitoring flow meter and are monitored respectively by associated data processors and the data processor of the mixing device.

3. A mixing installation as claimed in Claim 1 or 2, **characterised in that** the measuring chamber (9) has an upper, transparent section, at which the filling level may be determined with optoelectronic means provided there.

4. A mixing installation as claimed in Claim 1 or 2, **characterised in that** a capacitive filling level sensor (16) is inserted into the measuring chamber (9).

5. A mixing installation as claimed in one of Claims 1 to 4, **characterised in that** the measuring chamber is provided with an electrolytic ozone cell (32).

6. A mixing installation as claimed in one of Claims 1 to 5, **characterised in that** the mixing device further includes means for monitoring the conductivity of the finished solution.

7. A mixing installation as claimed in one of Claims 1 to 6, **characterised in that** the mixing device further includes means for monitoring the temperature of the finished solution.

8. A mixing installation as claimed in one of Claims 1 to 7, **characterised in that** the feedstock container (37) has a lower, tangential inlet (77) and an upper, tangential outlet (78).

9. A mixing installation as claimed in Claim 8, **characterised in that** the feedstock container has a further, lower, tangential outlet (76) of smaller diameter, which communicates with the upper outlet conduit via a conduit disposed outside the feedstock container (37).

10. A mixing installation as claimed in one of Claims 1 to 9, **characterised in that** the feedstock container (37) has a closable cover with an inwardly extending, central, conical-shaped formation, which fills the free space in the container (37) during the mixing process.

11. A mixing installation as claimed in one of Claims 1 to 10, **characterised in that** the recirculation circuit is provided with at least one compressed air connection in order to empty the conduits and the containers (36/37/44, 44a).

## Revendications

1. Installation de mélange servant à produire des solutions propres à l'usage médical avec une source d'eau extra-pure, qui est reliée par l'intermédiaire d'une conduite d'arrivée (1) à une conduite d'arrivée d'un appareil de mélange,
sachant que l'appareil de mélange contient un ordinateur et un circuit de recirculation, dans lequel sont installés une pompe (6), un réservoir de matières brutes (36, 37) contenant avant le début de l'opération de mélange un concentré liquide, pâteux, pulvérulent ou granulé, et un réservoir de solution prête à l'emploi (44) pouvant être rempli avant le début de l'opération de mélange d' un volume défini d'eau extra-pure,
**caractérisée en ce que** :
au moins un débitmètre (3, 4) relié à l'ordinateur associé est installé dans la conduite d'arrivée (1) de la source d'eau extra-pure ou de l'appareil de mélange, **en ce qu'**une chambre de mesure (9) volumtérique est disposée en aval dudit débitmètre, le niveau de remplissage de la chambre de mesure (9) étant déterminé, sachant que la valeur déterminée est comparée par l'ordinateur de l'appareil de mélange à la valeur de mesure du débitmètre au moins au nombre de un et que la valeur de mesure est au besoin corrigée,
et **en ce que** la chambre de mesure (9) est pourvue d'un capteur de densité à ultrasons (10) servant à mesurer la densité du liquide.

2. Installation de mélange selon la revendication 1,
**caractérisée en ce que** :
deux débitmètres (3, 4) sont disposés l'un derrière l'autre, lesquels fonctionnent de manière redondante en tant que débitmètre de service et débitmètre de contrôle et sont contrôlés respectivement par des ordinateurs associés et par l'ordinateur de l'appareil de mélange.

3. Installation de mélange selon la revendication 1 ou 2,
**caractérisée en ce que** :
que la chambre de mesure (9) comporte une section supérieure transparente, au niveau de laquelle le niveau de remplissage peut être détecté à l'aide de moyens optoélectroniques disposés à cet endroit.

4. Installation de mélange selon la revendication 1 ou 2,
**caractérisée en ce que** :
un capteur capacitif du niveau de remplissage (16) est introduit dans la chambre de mesure (9).

5. Installation de mélange selon l'une quelconque des revendications 1 à 4,
**caractérisée en ce que** :
la chambre de mesure est pourvue d'une cellule d'ozone (32) électrolytique.

6. Installation de mélange selon l'une quelconque des revendications 1 à 5,
**caractérisée en ce que** :
l'appareil de mélange présente en outre des moyens servant à contrôler la conductivité de la solution prête à l'emploi.

7. Installation de mélange selon l'une quelconque des revendications 1 à 6,
**caractérisée en ce que** :
l'appareil de mélange présente en outre des moyens servant à contrôler la température de la solution prête à l'emploi.

8. Installation de mélange selon l'une quelconque des revendications 1 à 7,
**caractérisée en ce que** :
le réservoir de matières brutes (37) comporte une arrivée (77) inférieure tangentielle et une évacuation (78) supérieure tangentielle.

9. Installation de mélange selon la revendication 8,
**caractérisée en ce que** :
le réservoir de matières brutes comporte une évacuation supplémentaire (76) inférieure tangentielle présentant un diamètre de plus petite taille, laquelle évacuation débouche dans la conduite d'évacuation supérieure par l'intermédiaire d'une conduite disposée à l'extérieur du réservoir de matières brutes (37).

10. Installation de mélange selon l'une quelconque des revendications 1 à 9,
**caractérisée en ce que** :
le réservoir de matières brutes (37) présente un couvercle refermable doté d'un épaulement central de forme conique faisant saillie vers l'intérieur, lequel remplit l'espace libre dans le réservoir (37) lors de l'opération de mélange.

11. Installation de mélange selon l'une quelconque des revendications 1 à 10,
**caractérisée en ce que** :
le circuit de recirculation est pourvu d' au moins un raccord d'air comprimé afin de vider les conduites et les réservoirs (36/37/44, 44a).
